(19) Europäisches Patentamt
European Patent Office
Office européen des brevets

(11) **EP 4 317 976 A1**

(12) **EUROPEAN PATENT APPLICATION**
published in accordance with Art. 153(4) EPC

(43) Date of publication:
**07.02.2024 Bulletin 2024/06**

(21) Application number: **22775806.7**

(22) Date of filing: **24.03.2022**

(51) International Patent Classification (IPC):
**G01N 33/543** (2006.01)

(52) Cooperative Patent Classification (CPC):
**G01N 33/543**

(86) International application number:
**PCT/JP2022/014157**

(87) International publication number:
**WO 2022/203025 (29.09.2022 Gazette 2022/39)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **26.03.2021 JP 2021052926**

(71) Applicant: **Denka Company Limited
Tokyo 103-8338 (JP)**

(72) Inventors:
• **AOYAMA Shuhei
Tokyo 103-8338 (JP)**
• **AKIYAMA Yuto
Tokyo 103-8338 (JP)**

(74) Representative: **Schnappauf, Georg
ZSP Patentanwälte PartG mbB
Hansastraße 32
80686 München (DE)**

(54) **INSPECTION DEVICE AND INSPECTION METHOD**

(57) A test device (100) includes: a flow path (103) provided on one surface of a sheet substrate (101) for transferring a liquid sample by a capillary phenomenon; a detection zone (105) provided in a portion of the flow path (103); and a fine rough structure A having a plurality of convex portions, the fine rough structure A being provided at least in the detection zone (105) and formed integrally with the sheet substrate (101) in the flow path (103), in which in the detection zone (105), a detection substance that specifically binds to a substance to be detected in the liquid sample is immobilized on a surface of the fine rough structure A, and the test device is configured to detect the substance to be detected by sensing a change in optical characteristics of the detection zone (105) occurring due to an enzymatic reaction.

FIG. 1

**Description**

TECHNICAL FIELD

The present invention relates to a test device and a test method.

BACKGROUND ART

As a technology for detecting a specific component in a sample, a technology described in Patent Document 1 (PCT Japanese Translation Patent Publication No. 2005-502363) is available. In the same document, a method for determining the presence of an analyte in a sample is described. Such a method specifically includes: providing a test device into which a lateral flow test strip that includes a dry matrix material capable of transferring a liquid by a capillary phenomenon and has at least an initiation area for receiving a sample and a reaction area having at least one immobilized enzyme; causing a movement of a liquid phase passing through the test strip by bringing the sample into contact with the initiation area; and determining a detectable signal that is directly or indirectly induced in the reaction area by covalent modification of an analyte brought by an enzyme in the reaction area.

RELATED DOCUMENT

PATENT DOCUMENT

[Patent Document 1] PCT Japanese Translation Patent Publication No. 2005-502363

SUMMARY OF THE INVENTION

TECHNICAL PROBLEM

When the inventors of the present invention examined the technology described in Patent Document 1, it became clear that there was room for improvement in terms of improving the detection accuracy.
The present invention provides a test device having excellent detection accuracy.

SOLUTION TO PROBLEM

According to the present invention, the following test device and test method are provided.

[1] A test device including:

a flow path provided on one surface of a sheet-shaped resin substrate for transferring a liquid sample by a capillary phenomenon;
a detection zone provided in a portion of the flow path; and
a fine rough structure A having a plurality of convex portions, the fine rough structure A being provided at least in the detection zone and formed integrally with the resin substrate,
in which in the detection zone, a detection substance that specifically binds to a substance to be detected in the liquid sample is immobilized on a surface of the fine rough structure A, and
the test device is configured to detect the substance to be detected by sensing a change in optical characteristics of the detection zone occurring due to an enzymatic reaction.

[2] The test device according to [1], in which the detection substance is an antibody against the substance to be detected.
[3] The test device according to [1] or [2], in which the change in the optical characteristics is a color change in the detection zone.
[4] The test device according to [3], in which the color change is visually identifiable.
[5] The test device according to [1] or [2], in which the change in the optical characteristics is chemiluminescence, bioluminescence, or fluorescence in the detection zone.
[6] The test device according to any one of [1] to [5], in which in the detection zone, the convex portions have a shape of a columnar body, a pyramidal body, or a frustum.
[7] The test device according to any one of [1] to [6], in which in the detection zone, the shape of the convex portions is a conical body, and the detection substance is immobilized on a lateral face of the conical body.

[8] The test device according to any one of [1] to [7], in which in the detection zone, the plurality of convex portions are planarly arranged in a lattice pattern.

[9] The test device according to any one of [1] to [8], in which the convex portions have a bottom surface with a diameter of equal to or more than 10 um and equal to or less than 1000 um.

[10] The test device according to any one of [1] to [9], in which the convex portions have a height of equal to or more than 10 um and equal to or less than 500 um.

[11] The test device according to any one of [1] to [10], in which at least in the detection zone, a fine rough structure B is formed on a surface of the convex portions, and

an arithmetic mean roughness Ra of a roughness curve of the convex portions on which the fine rough structure B is formed is equal to or more than 0.005 um and equal to or less than 1 um.

[12] The test device according to any one of [1] to [11], in which in one cross-section, a plurality of the convex portions have:

a first region located on one side of each convex portion from a center of the convex portion in a width direction of the convex portion; and

a second region located on other side of the convex portion from the center of the convex portion in the width direction of the convex portion, and

in the one cross-section, at least one of an outer edge of the first region and an outer edge of the second region of the convex portion has a concave portion.

[13] The test device according to any one of [1] to [12], which is used for an enzyme immunoassay method.

[14] A test method of using the test device according to any one of [1] to [13], the test method including:

a step of introducing the liquid sample into an upstream side of the detection zone to guide the liquid sample to the detection zone by a capillary phenomenon, and trapping the substance to be detected in the fine rough structure A by a specific interaction between the substance to be detected and the detection substance;

after the step of trapping, a step of introducing a liquid including a labeled antibody that specifically binds to the substance to be detected into the detection zone, and trapping the labeled antibody in the fine rough structure A in which the substance to be detected is trapped;

after the step of trapping the labeled antibody, a step of introducing, into the detection zone, a compound that causes a change in optical characteristics in the detection zone by reacting with the labeled antibody; and

a step of detecting or quantitatively determining the substance to be detected by sensing the change in the optical characteristics in the detection zone.

ADVANTAGEOUS EFFECTS OF INVENTION

According to the present invention, a test device having excellent detection accuracy can be provided.

BRIEF DESCRIPTION OF THE DRAWINGS

[Fig. 1] A top view showing a configuration example of a test device according to an embodiment.

[Fig. 2] Diagrams showing an example of the configuration of a detection zone of the device according to the embodiment.

[Fig. 3] Diagrams showing a SEM image of a fine rough structure A of the device according to Examples.

[Fig. 4] A top view showing an example of the arrangement of convex portions when the distance between adjacent convex portions is zero in the embodiment.

[Fig. 5] A diagram for explaining a concave portion on the outer edge of the first region of a convex portion in the embodiment.

[Fig. 6] Diagrams showing the results of an immunoassay using the device.

[Fig. 7] Diagrams showing the results of an immunoassay using the device.

[Fig. 8] Diagrams showing a SEM image of the convex portions of the device in the Examples.

[Fig. 9] Diagrams showing a SEM image of the convex portions of the device in the Examples.

[Fig. 10] Diagrams showing the results of an immunoassay using the device.

[Fig. 11] Diagrams showing an example of the configuration of the detection zone of the device according to the embodiment.

[Fig. 12] Diagrams showing an example of the configuration of the detection zone of the device according to the Examples.

[Fig. 13] A diagram showing a SEM image of the fine rough structure A of the device according to the Examples.

DESCRIPTION OF EMBODIMENTS

Hereinafter, embodiments of the present invention will be described by using the drawings. The drawings are schematic diagrams and are not consistent with the actual dimensional ratios.

In the present embodiment, a composition may include each component singly or as a combination of two or more kinds thereof.

In the present specification, the term "to" expressing a numerical value range means "equal to or more than" and "equal to or less than", and includes all the numerical values at both ends of the numerical value range.

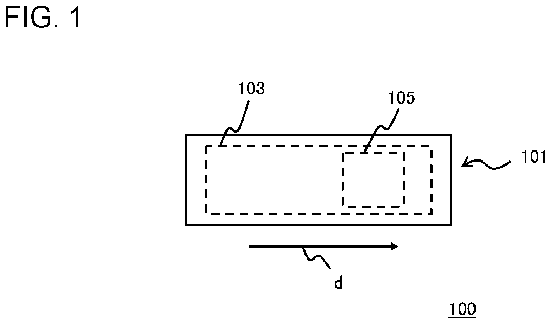

Fig. 1 is a top view showing a configuration example of a test device according to the present embodiment. The test device 100 shown in Fig. 1 has a flow path 103 provided on one surface of a sheet-shaped resin substrate (sheet substrate 101) for transferring a liquid sample by a capillary phenomenon; a detection zone 105 provided in a portion of the flow path 103; and a fine rough structure A having a plurality of convex portions, the fine rough structure A being provided at least in the detection zone 105 and formed integrally with the sheet substrate 101 in the flow path 103. In the detection zone 105, a detection substance that specifically binds to a substance to be detected in the liquid sample is immobilized on the surface of the fine rough structure A. The test device 100 is also configured to detect the substance to be detected by sensing a change in the optical characteristics of the detection zone 105 occurring due to an enzymatic reaction.

The test device 100 is specifically a lateral flow immunoassay device. Also, the test device 100 is suitable for, for example, an enzyme immunoassay method.

(Sheet Substrate)

The sheet substrate 101 is specifically a substrate used as a base material of the test device 100. Examples of the shape of the sheet substrate 101 include a sheet shape and a plate shape.

In the sheet substrate 101, as shown in the example of Fig. 1, the flow path 103 may be provided in some regions when seen in a plan view, or the flow path 103 may be provided over the entire sheet substrate 101.

Specific examples of the planar shape of the sheet substrate 101 include a polygon such as a quadrangle, a circle, and an ellipse. When the sheet substrate 101 is rectangular, the vertical width (length in the lateral direction) of the sheet substrate 101 may be, for example, about 1 to 100 mm, and the horizontal width (length in the longitudinal direction) of the sheet substrate 101 may be, for example, about 2 to 100 mm.

From the viewpoint of improving the strength of the test device 100, the thickness of the sheet substrate 101 is, for example, equal to or more than 0.05 mm, and preferably equal to or more than 0.1 mm.

From the viewpoint of thinning the test device 100, the thickness of the sheet substrate 101 is, for example, equal to or less than 5 mm, and preferably equal to or less than 3 mm.

Specifically, the material of the sheet substrate 101 may be one kind or two or more kinds selected from the group consisting of resin materials, such as poly(meth)acrylate such as polymethyl (meth)acrylate, polyester, polyolefin, polystyrene, polycarbonate, fluororesin, polyvinyl chloride, polyamide, and polyimide.

From the viewpoint of stably and integrally molding the sheet substrate 101 and a plurality of convex portions, the material of the sheet substrate 101 is preferably a thermoplastic resin. The thermoplastic resin may be specifically one kind or two or more kinds selected from the group consisting of polyester, polyolefin, polystyrene, polycarbonate, fluororesin, and (meth)acrylic resin, and may be more specifically one kind or two or more kinds selected from the group consisting of polyethylene terephthalate (PET), cycloolefin polymer (COP), polypropylene (PP), polystyrene (PS), polycarbonate (PC), polyvinylidene fluoride (PVDF), polymethyl methacrylate (PMMA), and polyethylene (PE).

(Detection zone)

The detection zone 105 is provided with a fine rough structure A. The fine rough structure A has a plurality of convex portions.

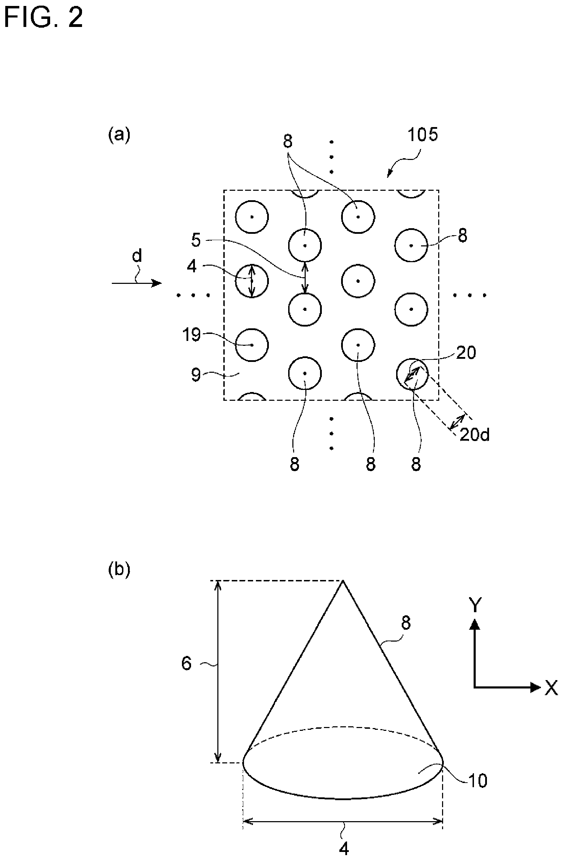

Fig. 2(a) and Fig. 2(b) are diagrams showing a configuration example of the fine rough structure A provided on the sheet substrate 101 and the convex portions constituting the fine rough structure A. Fig. 2(a) is a top view showing an example of the arrangement of convex portions 8 in the detection zone 105, and Fig. 2(b) is a perspective view showing an example of the shape of a convex portion 8.

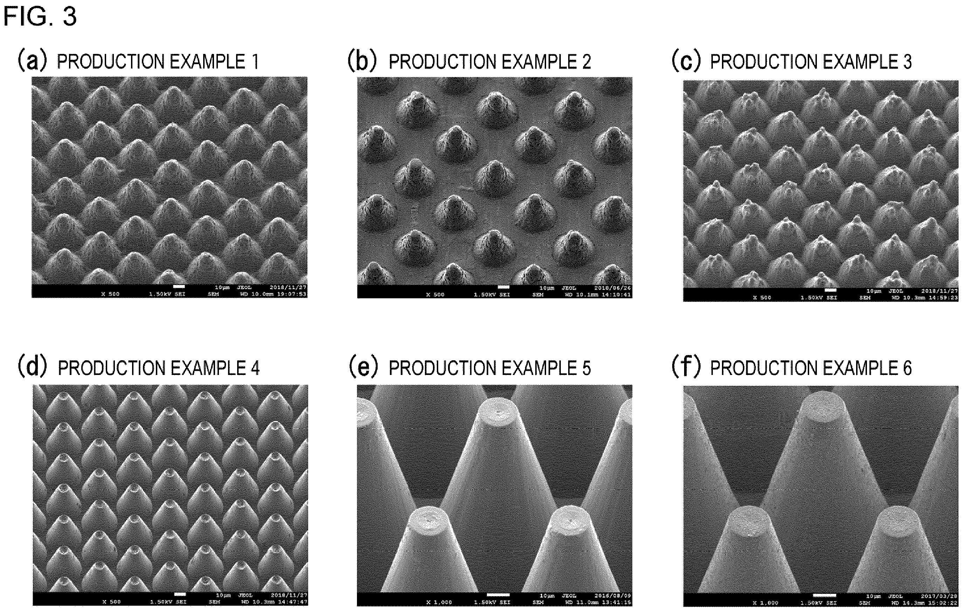

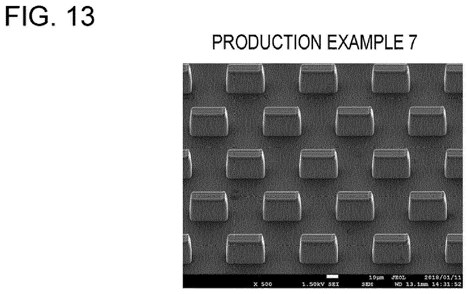

Fig. 3(a) to Fig. 3(f) and Fig. 13 are perspective views (SEM images) showing configuration examples of the fine rough structure A obtained in the section of Examples that will be described later.

In the detection zone 105, as shown in Fig. 2(a) and Fig. 2(b), the sheet substrate 101 includes a flat portion 9 corresponding to a bottom surface of the flow path 103, and a plurality of convex portions 8 protruding from the flat portion 9, and the fine rough structure A is formed by the whole of the plurality of convex portions 8. A liquid sample is transferred from the side of sample introduction on the left side of the drawing toward the side of sample discharge through the fine rough structure A, specifically along the transfer direction d in Fig. 1 and Fig. 2(a). In the example of Fig. 1, the transfer direction d is the longitudinal direction of the sheet substrate 101.

The spaces between the plurality of convex portions 8 function as the flow path 103 for transferring the liquid sample along the surface of the sheet substrate 101. In other words, voids in the fine rough structure A function as the flow path 103 that transfers the liquid sample along the surface of the sheet substrate 101.

As shown in Fig. 2(a) and Fig. 3(a) to Fig. 3(f), the plurality of convex portions 8 are preferably planarly arranged in a lattice pattern. More specific examples of the arrangement in a lattice pattern include a square lattice; and an oblique lattice such as a hexagonal lattice.

Specific examples of the shape of the convex portions 8 include columnar bodies such as a cylinder and a polygonal prism; pyramidal bodies such as a cone and a polygonal pyramid; and frustums such as a truncated cone and a truncated pyramid. These do not need to be geometrically accurate shapes and may be shapes having rounded corners or shapes having fine unevenness on the surface.

From the viewpoint of fabricating the convex portions 8 with higher reproducibility and from the viewpoint of improving the measurement accuracy for the substance to be detected, the shape of the convex portions 8 is more preferably a cone or a truncated cone, and even more preferably a conical body. From the same viewpoint, the shape of the convex portions 8 is still more preferably a conical body, and a detection substance is immobilized on the lateral face of such conical bodies. Fig. 2(b), Fig. 3(a), and Fig. 3(b) show examples in which the convex portions 8 are conical bodies. Fig. 3(c) and Fig. 3(d) are examples of conical-shaped convex portions 8 having unevenness formed at the apexes and conical-shaped convex portions 8 having their apexes formed in a concaved shape, respectively. Furthermore, Fig. 3(e) and Fig. 3(f) are examples of convex portions 8 having a truncated cone shape.

The shapes of the plurality of convex portions 8 may be the same or different. From the viewpoint of fabricating a desired fine rough structure A with higher reproducibility, it is preferable that the plurality of convex portions 8 have the same shape.

When the bottom surface of a convex portion 8 (bottom surface 10 in Fig. 2(b)) has a circular planar shape, the diameter of the bottom surface of the convex portion 8 is preferably equal to or more than 10 um, and more preferably equal to or more than 20 um, from the viewpoint of improving formability.

Furthermore, from the viewpoint of improving formability, the diameter of the bottom surface of a convex portion 8 (diameter 4 in Fig. 2(a) and Fig. 2(b)) is preferably equal to or less than 1000 um, and more preferably equal to or less than 500 um.

The height of the convex portion 8 is preferably equal to or more than 10 um, and more preferably equal to or more than 20 um, from the viewpoint of improving the measurement accuracy for the substance to be detected.

From a similar viewpoint, the height of the convex portions 8 (height 6 in Fig. 2(b)) is preferably equal to or less than 500 um, more preferably equal to or less than 200 um, and even more preferably equal to or less than 100 um.

The distance between adjacent convex portions 8 (distance 5 in Fig. 2(a)), that is, the distance of closest approach between convex portions 8, is appropriately set according to the shape of the convex portions 8 and is, for example, about 0 to 500 um.

For example, when the convex portions 8 are columnar bodies, the above-described distance of closest approach is preferably more than 0 um, and when the convex portions 8 are pyramidal bodies or frustums, the distance of closest approach is equal to or more than 0 um. Furthermore, the distance of closest approach may be, for example, equal to or more than 0.1 um, and may also be, for example, equal to or more than 1 um.

The upper limit of the distance of closest approach is preferably equal to or less than 500 pm, more preferably equal to or less than 100 pm, even more preferably equal to or less than 50 pm, and still more preferably equal to or less than 10 $\mu$m. As a result, the contact area between the liquid sample and the flow path 103 is increased, followed by an increase in the capillary force, and therefore, it becomes easy to transfer the liquid sample.

Here, the "distance between adjacent convex portions 8" is a distance defined as the distance between the peripheral surfaces of two convex portions 8 located closest to each other when seen in a top view, on a line segment connecting the center points of the two convex portions 8.

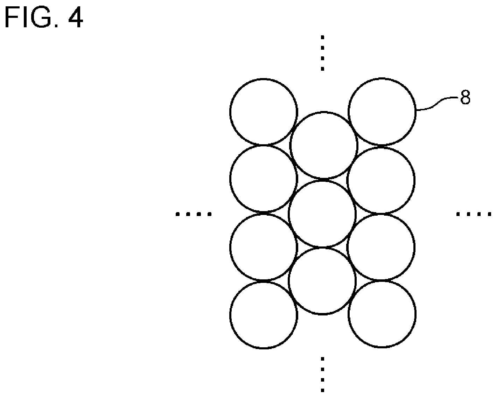

Fig. 4 is a top view showing an arrangement example of the convex portions 8 when the distance between adjacent convex portions 8 is zero. Fig. 4 shows an example in which the shape of the bottom surface of a convex portion 8 is a circle, and in this case, the convex portion 8 specifically has a shape of a conical body or a truncated cone. As shown in Fig. 4, for example, when the distance between adjacent convex portions 8 that are arranged in a hexagonal lattice is zero, a plurality of convex portions 8 are aligned without gaps between the bottom surfaces thereof, the number of convex portions 8 per unit area in a top view increases, the capillary force is further increased, and therefore, it becomes easier to transfer the liquid sample.

Here, each numerical value related to the size of the convex portions 8 is calculated by, for example, observing a cross-section of the convex portions 8 perpendicular to the flat portion 9 by using a scanning electron microscope (SEM), or observing a three-dimensional image of the convex portions 8 by using an optical microscope.

Furthermore, from the viewpoint of improving the measurement accuracy of the substance to be detected, it is also preferable that a fine rough structure B is formed on the surface of the convex portions 8 at least in the detection zone 105.

From the same viewpoint, the arithmetic mean roughness Ra of a roughness curve of the convex portions 8 on which the fine rough structure B is formed is preferably equal to or more than 0.005 um and equal to or less than 1 um.

Here, the arithmetic mean roughness Ra of the roughness curve is specifically measured according to JIS B 0601:2013 by using a three-dimensional roughness analysis scanning electron microscope.

From the viewpoint of immobilizing the detection substance more stably, the arithmetic mean roughness Ra of the roughness curve of the convex portions 8 on which the fine rough structure B is formed is preferably equal to or more than 0.005 um, more preferably equal to or more than 0.010 um, even more preferably equal to or more than 0.050 um, and still more preferably equal to or more than 0.080 um.

From the viewpoint of improving the formability of the fine rough structure B, the arithmetic mean roughness Ra is preferably equal to or less than 1 um, more preferably equal to or less than 0.5 um, even more preferably equal to or less than 0.3 um, and still more preferably equal to or less than 0.2 um.

In the fine rough structure A, the method for measuring the arithmetic mean roughness Ra of the convex portions 8 having the fine rough structure B formed will be described below by using Fig. 2(a) as an example.

Using a three-dimensional roughness analysis scanning electron microscope, a convex profile is measured along the surface of the convex portions 8 (along a straight line 20 when viewed from the top face), by taking the center of the apex of a convex portion 8 (for example, center 19 of the protruding part) as a center point. The straight line 20 is a single straight line having a length 20d, which has the center of the apex (for example, the center 19 of a convex portion 8) as a center point. The length 20d is the same length as the diameter of the bottom surface of the convex portion 8. When the straight line 20 is a straight line on the same plane (for example, when both ends and the center are on the same plane), that is, when the convex portions 8 have a shape of a cylinder, a polygonal prism, or the like, the arithmetic mean roughness Ra of the roughness curve defined by JIS B 0601:2013 is calculated from a concavo-convex profile. When the straight line 20 is not a straight line on the same plane, that is, when the convex portions 8 have a shape of a cone, a polygonal pyramid, a hemisphere, a hemi-ellipsoid, a truncated cone, a truncated pyramid, or the like, the arithmetic mean roughness Ra of the roughness curve defined by JIS B 0601:2013 is calculated by subjecting the concavo-convex profile to tilt correction to make it as a plane.

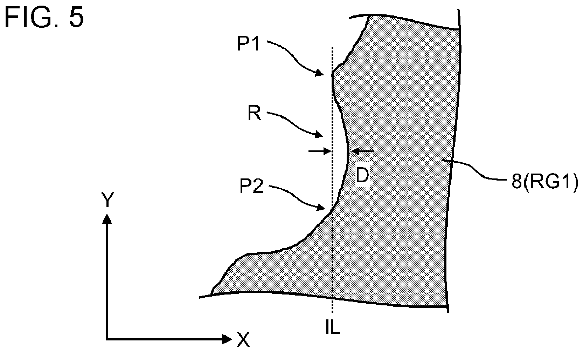

Furthermore, it is also preferable that the convex portion 8 has the following configuration. That is, it is also preferable that in one cross-section, a plurality of convex portions 8 have: a first region (RG1 in Fig. 5) located on one side of the convex portion 8 from a center of the convex portion 8 in a width direction of the convex portion 8; and a second region located on the other side of the convex portion 8 from the center of the convex portion 8 in the width direction of the convex portion 8, and that in the one cross-section, at least one of the outer edge of the first region and the outer edge of the second region of the convex portion 8 has a concave portion.

Fig. 5 is a diagram for explaining a concave portion (recess) R on the outer edge in the first region RG1 of a convex portion 8. Fig. 5 corresponds to an enlarged view of the outer edge of the convex portion 8 shown in Fig. 2(b). In Fig. 5, the X-direction indicates a direction parallel to the flat portion 9 (Fig. 2(a)). The Y-direction indicates a direction perpendicular to the flat portion 9.

The outer edge of the first region RG1 of the convex portion 8 has a concave portion R. The concave portion R is concaved (recessed) by a distance D in the X-direction with respect to an imaginary straight line IL. The distance D is, for example, equal to or more than 50 nm and equal to or less than 500 nm. In Fig. 5, the imaginary straight line IL extends in the Y-direction. Specifically, the imaginary straight line IL is in contact with a first position P1 on the outer edge of the convex portion 8 and passes through a second position P2. The first position P1 on the outer edge of the convex portion 8 is a position located on the outermost side of the convex portion 8 in the upper end part of a concave portion R. Therefore, the imaginary straight line IL is in contact with the first position P1. That is, at the first position P1 and in the vicinity thereof, the imaginary straight line IL and the outer edge of the convex portion 8 geometrically share only the first position P1 (that is, one point). A second position P2 on the outer edge of the convex portion 8 is located below the first position P1 in the Y-direction.

Fig. 5 shows a single concave portion R in a portion of the outer edge in the first region RG1 of the convex portion 8. The number of concave portions R in the entire first region RG1 is, for example, equal to or more than 1, and preferably equal to or more than 2, and the number is, for example, equal to or less than 20, preferably equal to or less than 10, and more preferably equal to or less than 5.

The concave portions on the outer edge in the second region of the convex portion 8 are also determined in the same manner as in the method shown in Fig. 5.

In a membrane carrier for a test kit according to the present embodiment, as the number of concave portions in the first region or the second region (for example, the concave portions R in the first region RG1) is equal to or more than a certain number, the support amounts of the detection substance in the detection zone 105 as well as reaction product substances generated by enzyme-substrate reactions, other chemical reactions, and biological reactions, and coloring substances can be further increased, and as a result, the detection sensitivity of the substance to be detected in the liquid sample can be further improved.

The reason why the support amount of the detection substance in the detection zone 105 as well as the reaction product substances generated by enzyme-substrate reactions, other chemical reactions, and biological reactions, and the coloring substances can be increased is not clearly understood; the following reasons may be considered.

First, the matter that the number of concave portions in the first region or the second region is equal to or more than a certain number serves as an indicator indicating that the surface area of protruding parts is increased due to the presence of the concave portions.

Therefore, it is speculated that as the number of concave portions in the first region or the second region is equal to or more than a certain number at least in the detection zone 105, a structure having a space suitable for supporting the detection substance as well as the reaction product substances generated by enzyme-substrate reactions, other chemical reactions, and biological reactions, and the coloring substances is formed. Therefore, in regard to the membrane carrier for a test kit according to the present embodiment, it is speculated that as the number of concave portions in the first region or the second region is equal to or more than a certain number at least in the detection zone 105, the support amounts of the detection substance in the detection zone 105 as well as the reaction product substances generated by enzyme-substrate reactions, other chemical reactions, and biological reactions, and the coloring substances can be increased.

As described above, it is considered that in the membrane carrier for a test kit according to the present embodiment, the detection sensitivity for a substance to be detected in a liquid sample can be further improved as the number of concave portions in the first region or the second region is equal to or more than a certain number.

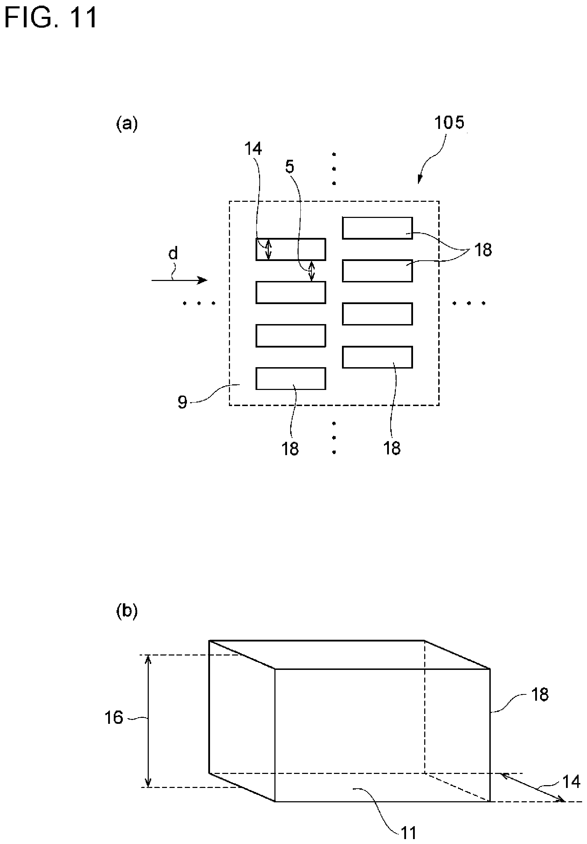

Fig. 11(a) is a top view showing an example of the planar arrangement of quadrangular prismatic convex portions, and Fig. 11(b) is a perspective view showing the shape of a convex portion 18 shown in Fig. 11(a).

As shown in Fig. 11(a) and Fig. 11(b), a fine rough structure A having a line-and-space structure, in which the shape of each convex portion 18 is a quadrangular prism and the convex portions 18 are linearly arranged, can also be provided.

When the planar shape of the convex portion 18 is a rectangle, the diameter 14 of the bottom surface 11 of the convex portion 18 is the length of the shortest side of the bottom surface (rectangle) 11 (in Fig. 11(a), length in a direction orthogonally intersecting the transfer direction d of the liquid sample). Furthermore, the height 16 of the convex portion 18 is the maximum length of the convex portion 18 in a direction orthogonally intersecting the flat portion 9 (height of the quadrangular prism).

In the detection zone 105, a detection substance is immobilized on the surface of the fine rough structure A. More specifically, the detection substance is selectively immobilized in the detection zone 105 in the flow path 103.

The detection substance may be chemically immobilized on the surface of the fine rough structure A or may be physically immobilized. From the viewpoint of retaining the detection substance more stably, the detection substance is preferably covalently bonded to the material of the sheet substrate 101 forming the fine rough structure A. At this time, the detection substance may be directly bonded to the material of the sheet substrate 101 in the fine rough structure A or may be bonded to intercalated molecules such as spacer molecules that are bonded to the material of the sheet substrate 101 in the fine rough structure A. It is preferable that the intercalated molecules include a glycol, an ether, an amine, an ester, an amide, an alcohol, a carboxylic acid, and the like.

The detection substance is selected from substances that specifically bind to the substance to be detected. For example, when the substance to be detected is an antigen, the detection substance may be an antibody against the antigen or an antigenbinding fragment thereof, and the detection substance is preferably an antibody against the substance to be detected. Here, the antigen may be a substance having immunogenicity by itself, or may be a hapten.

Furthermore, the substance to be detected may be a first antibody, and the detection substance may be a second antibody specific to the first antibody.

When the detection substance is an antibody or antigenic fragment thereof, the antibody may be a polyclonal antibody or may be a monoclonal antibody.

When the detection substance is an antibody or an antigenbinding fragment thereof, the substance to be detected may be a substance capable of undergoing an antigen-antibody reaction with the antibody, and examples include various pathogens and various clinical markers. More specific examples of the substance to be detected include virus antigens such as influenza virus, norovirus, adenovirus, RS virus, HAV, HBs, and HIV; bacterial antigens such as MRSA, group A streptococcus, group B streptococcus, and bacteria of the genus Legionella; toxins produced by bacteria and the like; Mycoplasma, Chlamydia trachomatis; hormones such as human chorionic gonadotropin; C-reactive protein, myoglobin, cardiac troponin, various tumor markers, agrochemicals, and environmental hormones. When the substance to be detected is an item requiring urgent detection and therapeutic measures, such as influenza virus, norovirus, C-reactive protein, myoglobin, or cardiac troponin, usefulness thereof is even more significant. The substance to be detected is usually in a state of being suspended or dissolved in the liquid sample. The liquid sample may be, for example, a sample in which the substance to be detected is suspended or dissolved in a buffer solution.

The substance to be detected may be selected from the group consisting of, for example, proteins and peptides such as enzymes and antibodies; nucleic acids and nuclear proteins; polysaccharides; and glycoproteins. The detection substance may be a substance having specificity for these substances to be detected. For example, the detection substance may be selected from the group consisting of proteins, nucleic acids, polysaccharides, and glycoproteins.

The test device 100 is configured so as to detect a substance to be detected by detecting a change in the optical characteristics in the detection zone 105 occurring due to an enzymatic reaction. For this reason, the test device 100 has excellent detection accuracy. Furthermore, according to the present embodiment, it is also possible to obtain, for example, a test device 100 having excellent detection accuracy and sensitivity.

A change in the optical characteristics is specifically a color change in the detection zone 105, or chemiluminescence, bioluminescence, or fluorescence in the detection zone 105.

When the change in the optical characteristics is a color change in the detection zone 105, the test device 100 is preferably configured such that the color change can be visually identifiable.

(Method for manufacturing test device)

Next, a method for manufacturing the test device 100 will be described.

The test device 100 can be obtained by, for example, forming a fine rough structure A in a predetermined region on one surface of the sheet substrate 101 and immobilizing a detection substance in the predetermined region of the region of formation of the fine rough structure A to form a detection zone 105.

Examples of the method of forming the fine rough structure A include imprinting such as thermal imprinting and UV imprinting;

injection molding;
pattern formation on a UV-curable resin by photolithography, soft lithography using a UV-curable resin having a pattern formed thereon by photolithography as a template, pattern formation by etching or the like by utilizing a UV-curable resin having a pattern formed thereon by photolithography;
machining; and
laser processing. Among these, as a technique of performing precision processing at low cost, thermal imprinting on a thermoplastic resin and injection molding are suitable. Specific examples of the thermoplastic resin include those described above as the material of the sheet substrate 101.

When the shape of the convex portions 8 in the fine rough structure A is a pyramidal body, in a processing method using a mold such as imprinting or injection molding, since the pyramidal body is tapering toward the top as compared with the bottom surface, the volume to be carved out during mold fabrication is smaller than the volume needed to fabricate a columnar body having the same bottom surface, and a mold can be fabricated at a low cost. In this case, it is possible to perform detection of the substance to be detected in the liquid sample at a lower cost.

Furthermore, the immobilization of the detection substance on the fine rough structure A can be performed, for example, by the following method. In an example where the detection substance is an antibody, for example, a method of applying an antibody solution on the fine rough structure A and incubating the fine rough structure A; and a method of applying either or both of a silane coupling agent and a crosslinking agent on the surface of the fine rough structure A, subsequently causing a reaction with the material of the sheet substrate 101, applying an antibody solution on the site of application, and incubating the fine rough structure A, may be mentioned.

The silane coupling agent for the latter method may be, for example, one kind or two or more kinds selected from the group consisting of a silane coupling agent having an amino group, a silane coupling agent having a mercapto group, a silane coupling agent having an epoxy group, a silane coupling agent having an acrylic group, a silane coupling agent having a methacryl group, a silane coupling agent having a vinyl group, and a silane coupling agent having an isocyanate group.

Examples of the silane coupling agent having an amino group include 3-aminopropyltriethoxysilane, 3-aminopropyltrimethoxysilane, 3-(2-aminoethyl)aminopropyltriethoxysilane, and 3-(2-aminoethyl)aminopropyltrimethoxysilane.

Examples of the silane coupling agent having a mercapto group include 3-mercaptopropyltrimethoxysilane, 3-mercaptopropyltriethoxysilane, 2-mercaptoethyltrimethoxysilane, and 2-mercaptoethyltriethoxysilane.

Examples of the silane coupling agent having an epoxy group include 3-glycidoxypropyltrimethoxysilane, 5,6-epoxyhexyltriethoxysilane, 2-(3,4-epoxycyclohexyl)ethyltrimethoxysilane, and 3-glycidoxypropyltriethoxysilane.

Examples of the silane coupling agent having an acrylic group include 3-acryloxypropyltrimethoxysilane, 3-acryloxypropylmethyldimethoxysilane, 3-acryloxypropylmethyldiethoxysilane, and 3-acryloxypropyltriethoxysilane.

Examples of the silane coupling agent having a methacryl group include 3-methacryloxypropylmethyldimethoxysilane, 3-methacryloxypropyltrimethoxysilane, 3-methacryloxypropylmethyldiethoxysilane, 3-methacryloxypropyltriethoxysilane, γ-(methacryloyloxypropyl)trimethoxysilane, and γ-(methacryloyloxypropylmethyl)dimethoxysilane.

Examples of the silane coupling agent having a vinyl group include vinyltriethoxysilane and vinyltrimethoxysilane.

Examples of the silane coupling agent having an isocyanate group include trimethoxysilylmethyl isocyanate, triethoxysilylmethyl isocyanate, tripropoxysilylmethyl isocyanate, 2-trimethoxysilylethyl isocyanate, 2-triethoxysilylethyl isocyanate, 2-tripropoxysilylethyl isocyanate, 3-trimethoxysilylpropyl isocyanate, 3-triethoxysilylpropyl isocyanate, 3-tripropoxysilylpropyl isocyanate, 4-trimethoxysilylbutyl isocyanate, 4-triethoxysilylbutyl isocyanate, and 4-tripropoxysilylbutyl isocyanate.

From the viewpoint of increasing the amount of the detection substance supported on the fine rough structure A, the silane coupling agent is preferably at least one kind selected from the group consisting of a silane coupling agent having an amino group and a silane coupling agent having an epoxy group.

Furthermore, the crosslinking agent may be one or two or more compounds selected from the group consisting of formaldehyde, glutaraldehyde, dextran, 1,4-phenyl diisocyanate, toluene-2,4 diisocyanate, polyethyleneimine, hexamethylene diisocyanate, hexamethylene diisothiocyanate, N, N'-polymethylene bisiodoacetamide, N, N'-ethylenebismaleimide, ethylene glycol bissuccinimidyl succinate, bisdiazobenzidine, 1-ethyl-3-(3-dimethylaminopropyl)carbodiimide, succinimidyl 3-(2-pyridyldithio)propionate, N-succinimidyl 4-(N-maleimidomethyl)cyclohexane-1-carboxylate, N-hydroxysucciimide, N-sulfosuccinimidyl 4-(N-maleimidomethyl)cyclohexane-1-carboxylate, N-succinimidyl (4-iodoacetyl)aminobenzoate, N-succinimidyl 4-(1-maleimidophenyl)butyrate, N-(ε-maleimidocaproyloxy)succinimide, iminothiolane, S-acetylmercaptosuccinic anhydride, methyl-3-(4'-dithiopyridyl)propionimidate, methyl-4-mercaptobutyrylimidate, methyl-3-mercaptopropionimidate, and N-succinimidyl-S-acetylmercaptoacetate.

From the viewpoint of increasing the amount of the detection substance supported on the fine rough structure A, the crosslinking agent is preferably one or two or more compounds selected from the group consisting of glutaraldehyde, dextran, 1,4-phenyl diisocyanate, toluene-2,4 diisocyanate, polyethyleneimine, 1-ethyl-3-(3-dimethylaminopropyl)carbodiimide, N-succinimidyl 4- (N-maleimidomethyl)cyclohexane-1-carboxylate, and N-hydroxysucciimide.

(Test method)

In the present embodiment, the test method using the test device 100 can be carried out by, for example, a sandwich method. In this case, the test method specifically includes:

> a step of introducing a liquid sample including a substance to be detected into the upstream side of the detection zone 105, guiding the liquid sample to the detection zone 105 by a capillary phenomenon, and trapping the substance to be detected into the fine rough structure A by a specific interaction between the substance to be detected and the detection substance (step 11);
> after the step of trapping, a step of introducing a liquid including a labeled antibody that specifically binds to the substance to be detected into the detection zone 105, and trapping the labeled antibody into the fine rough structure A in which the substance to be detected is trapped (step 12);
> after the step of trapping the labeled antibody, a step of introducing a compound that causes a change in the optical characteristics in the detection zone 105 by reacting with the labeled antibody, into the detection zone 105 (step 13); and
> a step of detecting or quantitatively determining the substance to be detected in the liquid sample by sensing a change in the optical characteristics in the detection zone 105 (step 14).

Furthermore, in at least one of before step 11, between step 11 and step 12, between step 12 and step 13, and between step 13 and step 14, a step of introducing a buffer solution or the like into the upstream side of the detection zone 105 and rinsing off the detection zone 105 (step 15) may be further carried out.

As the labeled antibody for step 12, those labeled antibodies used for immunoassays such as enzyme-linked immunosorbent assay (ELISA) and fluorescence immunoassay may be used. Examples of the labeled antibody include an enzyme-labeled antibody.

Step 13 is specifically a step of introducing a substrate for causing color development or luminescence into the detection zone 105. As the compound for step 13, a compound that reacts with a labeled antibody to produce an optical change may be used. Examples of the above-described compound include a chromogenic substrate and a luminescent substrate, both of which react with an enzyme-labeled antibody. Here, the luminescent substrate may be any of a chemiluminescent substrate, a bioluminescent substrate, and a fluorogenic substrate.

Furthermore, in step 14, specifically, the labeled antibody trapped in the fine rough structure A is detected. In step 14, a detection method suitable for the type of the labeled antibody used in step 12 can be used.

For example, in step 14, when the change in the optical characteristics is a color change or a luminescence change caused by a reaction between an enzyme and a substrate in the detection zone, the substance to be detected can be detected or quantitatively determined by measuring the presence or absence of color development, light absorbance, fluorescence, chemiluminescence, bioluminescence, or the like, all of which are based on the labeled antibody substrate in the detection zone 105 in step 14.

Specific examples of a combination of an enzyme-labeled antibody and a chromogenic substrate include, for example, an alkaline phosphatase (ALP)-labeled antibody with a mixture of 5-bromo-4-chloro-3-indolyl phosphate and nitroblue tetrazolium (BCIP-NBT); a horseradish peroxidase (HRP)-labeled antibody with a mixture of 3,3'-diaminobenzidine (DAB) and hydrogen peroxide ($H_2O_2$); and a horseradish peroxidase (HRP)-labeled antibody with a mixture of 3,3',5,5'-tetramethylbenzidine (TMB) and hydrogen peroxide ($H_2O_2$).

Furthermore, in step 14, when the change in the optical characteristics is fluorescence, chemiluminescence, or bioluminescence in the detection zone 105, the substance to be detected can be detected or quantitatively determined by introducing a substrate for the enzyme immobilized on the labeled antibody in the detection zone 105 and measuring the presence or absence of fluorescence or chemiluminescence based on the substrate, or the intensity of fluorescence, the intensity of chemiluminescence, or the intensity of bioluminescence in step 14.

Specific examples of a combination of an enzyme-labeled antibody and a fluorogenic substrate include a combination of an alkaline phosphatase (ALP)-labeled antibody and 1,3-dichloro-9,9'-dimethyl-acridine-2-one-7-yl phosphate (DDAO phosphate), a combination of an ALP-labeled antibody and ELF (registered trademark, Thermo Fisher Scientific, Inc.), a combination of an ALP-labeled antibody and AttoPhos (registered trademark, JBL Scientific, Inc.), a combination of an ALP-labeled antibody and 6,8-difluoro-4-methylumbelliferyl phosphate (DiFMUP), a combination of an ALP-labeled antibody and 4-methylumbelliferyl phosphate (MUP), and a combination of an ALP-labeled antibody and fluorescein diphosphate tetraammonium salt (FDP).

Specific examples of a combination of an enzyme-labeled antibody and a chemiluminescent substrate include a combination of a horseradish peroxidase (HRP)-labeled antibody with a mixture of luminol, aqueous hydrogen peroxide ($H_2O_2$), and an iodophenol compound; a combination of an ALP-labeled antibody and AMPPD (registered trademark, Tropix, Inc.); a combination of an ALP-labeled antibody and CDP-Star (trademark, $C_{18}H_{19}Cl_2O_7PNa_2$); a combination of an ALP-labeled antibody and CSPD (registered trademark, Tropix, Inc., $C_{18}H_{20}ClO_7PNa_2$); and a combination of an ALP-labeled antibody and Lumigen (registered trademark) PPD ($C_{18}H_{21}O_7PNa_2$).

Specific examples of a combination of an enzyme-labeled antibody and a bioluminescent substrate is a combination of a luciferase-labeled antibody and luciferin.

(Chip)

In the present embodiment, the test device 100 can also be used for a chip. Furthermore, the chip has the above-mentioned test device 100 according to the present embodiment. The chip may be composed of the test device 100 or may further have other members. Specific examples of the other members include a housing that accommodates or holds the test device 100, and a member that absorbs the liquid that has flowed through the test device 100.

Embodiments of the present invention have been described with reference to the drawings; however, these are merely examples of the present invention, and various configurations other than the above-described ones can also be adopted.

EXAMPLES

Hereinafter, the present embodiment will be specifically described with reference to Examples and Comparative Examples; however, the present embodiment is not intended to be limited to these Examples.

(Production Examples 1 to 7)

In the present examples, sheet substrates 101 having the outline structure shown in Fig. 1 were produced by the following procedure.

(Production Example 1) Production of sheet substrate 1

A polycarbonate sheet (manufactured by Teijin, Ltd., PC-2151, 50 mm × 50 mm, sheet thickness 200 um) was subjected to thermal imprinting, and a sheet substrate 1 (Production Example 1: Fig. 3(a)) in which conical convex portions 8 having a diameter of the bottom surface of a fine rough structure A (convex portions 8) (hereinafter, also referred to as "diameter of convex portions" or "diameter") of 30 um and a height of the fine rough structure A (convex portions 8) (hereinafter, also referred to as "height") of 30 um were arrayed in a hexagonal lattice pattern with a distance between the convex portions 8 of 2 um, was fabricated. The planar shape of the region of formation of the convex portions 8 in the sheet substrate 1 was set to 30 mm $\times$ 30 mm.

Here, when applying thermal imprinting, a laser-processed mold was used to form a fine rough structure B on the surface of the fine rough structure A (convex portions 8), and the number of concave portions R on the outer edge in the first region RG1 and the number of concave portions R on the outer edge of the second region RG2 of the convex portions 8 on which the fine rough structure B was formed were adjusted to the values shown in Table 1.

The method for processing the mold used for the production of the sheet substrate 1 is as follows.

An aluminum alloy flat plate was irradiated a plurality of times with pulsed light from a laser processing apparatus (ultrashort pulse laser processing machine R-200 manufactured by Tosei Electrobeam Co., Ltd., laser wavelength: 1552 nm, rated power output: 10 W, pulse: femtoseconds) to obtain a mold having conical-shaped concave portions corresponding to the convex portions 8.

(Production Example 2) Production of sheet substrate 2

A sheet substrate 2 (Production Example 2: Fig. 3(b)) was fabricated in accordance with the sheet substrate according to Production Example 1, except that the convex portions 8 were arrayed in a square lattice pattern, with the distance between the convex portions 8 set to 15 um (distance between the centers of cones 45 um).

(Production Example 3) Production of sheet substrate 3

A sheet substrate 3 (Production Example 3: Fig. 3(c)) was fabricated in accordance with the sheet substrate according to Production Example 1, except that a nickel flat plate was irradiated a plurality of times with pulsed light from a laser processing apparatus (ultrashort pulse laser processing machine R-200 manufactured by Tosei Electrobeam Co., Ltd., laser wavelength: 1552 nm, rated power output: 10 W, pulse: femtoseconds) to obtain a mold having conical-shaped concave portions corresponding to the convex portions 8, and the obtained mold was used for imprint processing.

(Production Example 4) Production of sheet substrate 4

A sheet substrate 4 (Production Example 4: Fig. 3(d)) was fabricated in accordance with the sheet substrate according to Production Example 1, except that a mold having conical-shaped concave portions corresponding to the convex portions 8 was obtained by subjecting an acrylic flat plate to machine cutting processing and then performing Ni electroforming twice, and the obtained mold was used for imprint processing.

(Production Example 5) Production of sheet substrate 5

A sheet substrate 5 (Production Example 5: Fig. 3(e)) was fabricated in accordance with the sheet substrate according to Production Example 1, except that a mold having conical-shaped concave portions corresponding to the convex portions 8 was obtained by subjecting an acrylic flat plate to machine cutting processing by setting the diameter of the bottom surface of the fine rough structure A (convex portions 8) to 60 um and the height of the fine rough structure A (convex portions 8) to 90 um and setting the distance between the convex portions 8 to 2 um and then performing Ni electroforming twice, and the obtained mold was used for imprint processing.

(Production Example 6) Production of sheet substrate 6

A sheet substrate 6 (Production Example 6: Fig. 3(f)) was fabricated in accordance with the sheet substrate according to Production Example 5, except that a mold having conical-shaped concave portions corresponding to the convex portions 8 was obtained by subjecting an acrylic flat plate to machine cutting processing, subsequently performing Ni electroforming twice, and further subjecting the acrylic flat plate to etching using an iron chloride solution, and the obtained mold was used for imprint processing.

(Production Example 7) Production of sheet substrate 7

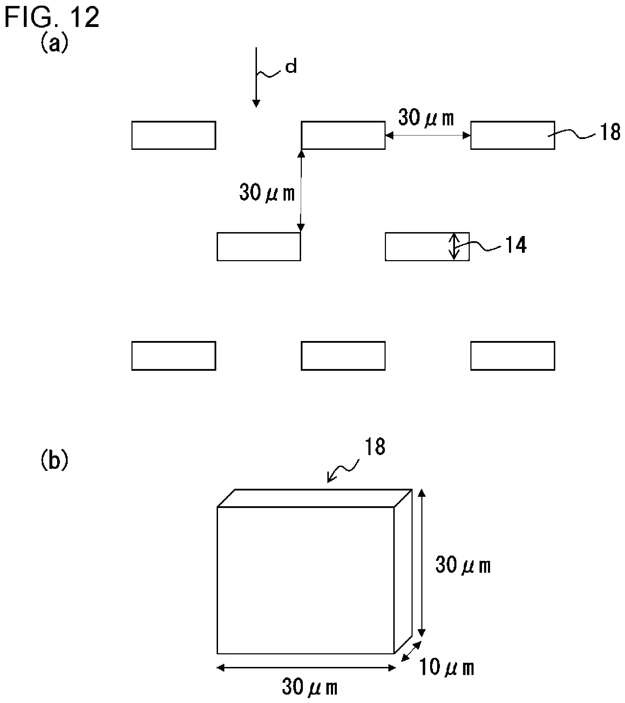

In the present example, a sheet substrate in which the fine rough structure A had an outline structure shown in Fig. 12(a) and Fig. 12(b) was produced. Fig. 12(a) is a top view showing the array of the convex portions 18 in the fine rough structure A, and Fig. 12(b) is a perspective view showing the shape of a convex portion 18. In the present example, a sheet substrate 7 (Production Example 7: Fig. 13) in which cuboid convex portions 18 having a size of 10 um (diameter 14 of the bottom surface) $\times$ 30 $\mu$m $\times$ 30 um (height) were arrayed as shown in Fig. 12(a), with the distance between the convex portions 18 set to 30 um, was fabricated.

The method for processing a mold used for the production of the sheet substrate 7 is as follows. A mold having cuboidshaped concave portions corresponding to the convex portions 18 was obtained by patterning concave portions on a glass substrate by photolithography and then performing Ni electroforming twice.

Fig. 3(a) to Fig. 3(f) and Fig. 13 are diagrams showing SEM images of the fine rough structure A of the sheet substrates obtained in Production Examples 1 to 7, respectively.

The measured values related to the size of the convex portions 8 of the sheet substrate obtained in each example are shown in Table 1.

[Table 1]

Table 1

| | Production Example 1 | Production Example 2 | Production Example 3 | Production Example 4 | Production Example 5 | Production Example 6 | Production Example 7 |
|---|---|---|---|---|---|---|---|
| Diameter of bottom surface of convex portions ($\mu$m) | 30 | 30 | 30 | 30 | 60 | 60 | $10 \times 30$ |
| Height of convex portions ($\mu$m) | 30 | 30 | 30 | 30 | 90 | 90 | 30 |
| Distance between convex portions ($\mu$m) | 2 | 15 | 2 | 2 | 2 | 2 | 30 |
| Arithmetic mean roughness Ra of roughness curve of convex portions ($\mu$m) | 0.114 | 0.120 | 0.078 | 0.014 | 0.0089 | 0.036 | 0.006 |
| Number of concave portions R on outer edge in RG1 | 3 | 3 | 15 | 1 | Not measured | Not measured | Not measured |
| Number of concave portions R on outer edge in RG2 | 4 | 3 | 13 | 1 | Not measured | Not measured | Not measured |

(Experimental Example 1: Example 1 and Comparative Example 1)

In Example 1 and Comparative Example 1, an anti-C-Reactive Protein (CRP) antibody as a detection substance was immobilized on the above-described sheet substrate 1 and a membrane substrate 1 (manufactured by Merck Millipore Corporation, thickness about 0.25 mm) formed from an unprocessed (not having a fine rough structure A) nitrocellulose membrane, respectively, to produce devices (Example 1: device 1, Comparative Example 1: device 2), and by using the obtained devices, CRP, an enzyme-labeled anti-CRP antibody, and a substrate were sequentially added dropwise thereto to perform an immunoassay by a sandwich method.

Specific procedures are shown below.

(Immobilization of Detection substance)

An anti-CRP antibody (manufactured by Bethyl Laboratories, Inc.) was added to a prepared buffer solution (composition: 50 mM trishydroxymethylaminomethane-hydrochloric acid buffer solution, pH 7.5, trehalose 2 (w/v)%) to prepare an antibody solution having an antibody concentration of about 0.1 ug/mL. In Example 1, the region of formation of the fine rough structure A in the sheet substrate 1 was cut into a size of 5 mm in width $\times$ 30 mm in length, while in Comparative Example 1, the membrane substrate 1 was cut into the same size, and the cut substrates were used for the device of each example. 1 $\mu$L of the prepared antibody solution was applied on each of the substrates at the center in the width direction of the substrate at a position 1.2 mm away from the upstream end of the substrate by using a micropipette, and then each substrate was dried at 42°C for 1 hour to fabricate a detection zone (sheet substrate 1: circular shape with a diameter of about 1 mm, membrane substrate 1: circular shape with a diameter of about 2 mm).

(Fabrication of Device)

Each of the sheet substrate 1 and the membrane substrate 1 having the antibody immobilized thereon was superposed with an end part (at a position 5 mm away from the downstream edge of the substrate) of an absorbent pad (PVA sponge, A-3150HP, manufactured by Aion Co., Ltd.) cut to a size of 5 mm in width $\times$ 50 mm in length, and the two were fixed by using a tape.

(Immunoassay)

An immunoassay was performed according to the following procedure by using the device obtained in each example. The immunoassay was performed by using a sample including an antigen or a sample including no antigen, with n=3 per condition for Example 1 and Comparative Example 1.

In the following procedures, unless stated otherwise, each aqueous solution was dropped onto the end part of the substrate of each example (at a position 5 mm away from the edge on the opposite side of the side superposed with the absorbent pad).

1. 20 $\mu$L of an aqueous solution for washing (0.05 (v/v)% Triton X-100 / Tris-buffered saline (TBS)) was dropped to wash surplus antibodies on the substrate of each example.

2. 20 $\mu$L of an aqueous solution obtained by diluting CRP (manufactured by BBI Solutions OEM, Ltd.) with 0.05 (v/v)% Triton X-100 / TBS (CRP concentration 10 ug/mL) or an aqueous solution including no CRP (0.05 (v/v)% Triton X-100 / TBS) was dropped, and the substrates were let stand for 3 minutes.

3. 20 $\mu$L of an alkaline phosphatase-labeled anti-CRP antibody solution (antibody concentration about 20 ug/mL) was dropped, and the substrates were let stand for 2 minutes. This operation was repeated once more.

4. 20 $\mu$L of a BCIP-NBT solution prepared by mixing a BCIP-NBT color-developing stock solution (manufactured by Nacalai Tesque, Inc.) with a Tris-HCl buffer solution (manufactured by Nacalai Tesque, Inc.) at a volume ratio of 1:100, was dropped, and the substrates were let stand for 3 minutes.

5. 20 $\mu$L of an aqueous solution for washing (0.05 (v/v)% Triton X-100 / TBS) was dropped to wash surplus antigens, the substrate, and the product material on the substrate.

6. Images of the substrate of each example after washing were captured by using a digital camera (manufactured by Casio Computer Co., Ltd., EXILIM EX-100F).

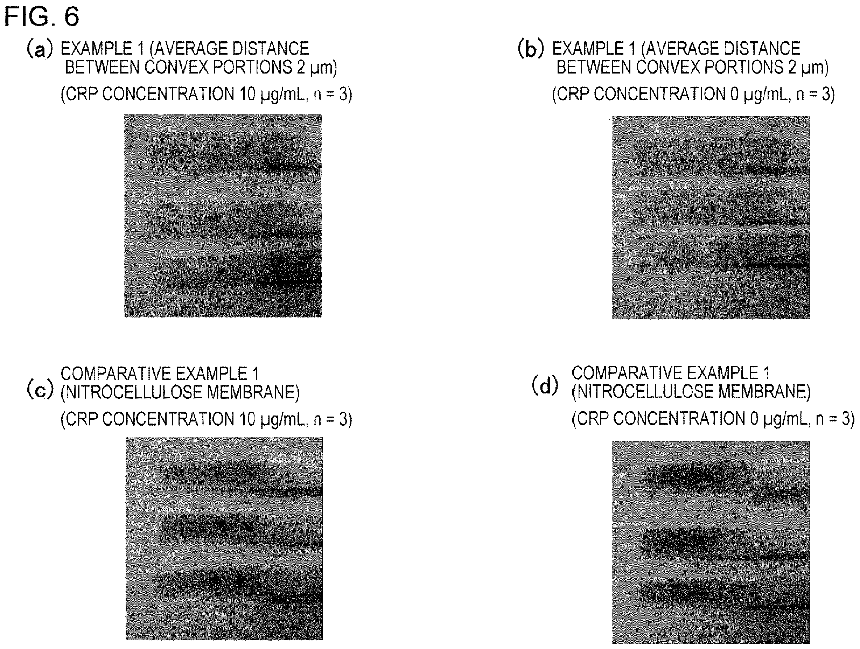

Images of the substrates obtained in the above described 6. are shown in Fig. 6(a) to Fig. 6(d). Fig. 6(a) and Fig. 6(b) are diagrams showing the results of dropping the CRP concentration of 10 ug/mL and the 0.05 (v/v)% Triton X-100 / TBS, respectively, in the above-described procedure 2. in Example 1. Fig. 6(c) and Fig. 6(d) are diagrams showing the results of dropping the CRP concentration of 10 ug/mL and the 0.05 (v/v)% Triton X-100 / TBS, respectively, in the above-described procedure 2. in Comparative Example 1.

From Fig. 6(a) to Fig. 6(d), purple coloration was identified on each substrate as a result of a reaction between the enzyme (alkaline phosphatase) introduced onto the substrate in the procedure 3. and the substrate (BCIP-NBT) introduced onto the substrate in the procedure 4. In the device 1 employing the sheet substrate having the fine rough structure A of Example 1, clear coloration was observed from the detection zone (region where the antibody was applied) of the substrate with which a sample containing the antigen was measured. Furthermore, coloration was hardly identified

EP 4 317 976 A1

in the regions other than the detection zone (regions where the antibody was not applied) of the substrate with which a sample containing the antigen was measured, and the substrate with which a sample containing no antigen was measured.

On the other hand, in the device 2 employing the membrane substrate of Comparative Example 1, coloration was observed not only in the region where the antibody was applied in the substrate with which a sample containing the antigen was measured, but also in the regions where the antibody was not applied and the substrate with which a sample containing no antigen was measured.

From the above-described results, it was found that in the device 1 employing the sheet substrate having the fine rough structure A of Example 1, since a detector substance and a coloring substance can be efficiently immobilized on the convex portions 8, a detection signal (coloration), which is a change in the optical characteristics caused by an enzymatic reaction, is easily recognized in the detection zone 105, that is, the S/N ratio is increased, as compared with the device 2 employing the membrane substrate of Comparative Example 1, and measurement with high sensitivity and high accuracy is enabled.

(Calculation of ΔRGB in region where antibody was applied)

For Example 1 and Comparative Example 1, images taken of the substrates of each example after the immunoassay were analyzed by using image analysis software, Image J (manufactured by the National Institutes of Health, USA), and the RGB values in the region where the antibody was applied were calculated. Specifically, the captured color image was separated into RGB images, and each of the R value, G value, and B value at the central part (circular shape with a diameter of 800 um) of the antibody-applied portion was calculated.

For the substrates of each example, ΔRGB was calculated according to the following formula by using the RGB values of the substrate with which a sample containing the antigen was measured, and the RGB values of the substrate with which a sample containing no antigen was measured.

[math. 1]

$$\Delta RGB = \sqrt{(R_n - R_0)^2 + (G_n - G_0)^2 + (B_n - B_0)^2}$$

In the above-described formula, $R_n$, $G_n$, and $B_n$ represent the R value, G value, and B value, respectively, for the substrate with which a sample containing the antigen was measured, and $R_0$, $G_0$, and $B_0$ represent the R value, G value, and B value, respectively, for the substrate with which a sample containing no antigen was measured.

The calculation results of ΔRGB for each substrate are shown in Table 2.

[Table 2]

Table 2

|  | Example 1 | Comparative Example 1 |
|---|---|---|
| ΔRGB n1 | 113.1 | 32.4 |
| ΔRGB n2 | 112.3 | 19.4 |
| ΔRGB n3 | 136.5 | 6.4 |
| ΔRGB Average | 120.6 | 19.4 |
| STD | 11.2 | 10.6 |
| CV Coefficient of variation (%) | 9.3 | 54.7 |

From Table 2, the device employing the sheet substrate having the fine rough structure A of Example 1 had a higher ΔRGB value as compared with the device employing the membrane substrate without the fine rough structure A of Comparative Example 1. Furthermore, with regard to the coefficient of variation of the measurement results of ΔRGB in three tests, the coefficient of variation of the device employing the sheet substrate having the fine rough structure A of Example 1 was smaller compared to the device using the membrane substrate of Comparative Example 1.

14

Therefore, it was found that compared to the device employing the membrane substrate of Comparative Example 1, the device employing the sheet substrate having the fine rough structure A of Example 1 can efficiently immobilize the detection substance and the coloring substance on the convex portions 8, and an antigen can be detected with higher sensitivity and higher accuracy.

(Experimental Example 2: Examples 2 to 8)

In Examples 2 to 8, an anti-CRP (C-Reactive Protein) antibody was immobilized as a detection substance on the above-mentioned sheet substrates 1 to 7, respectively, to produce devices (Example 2: device 3, Example 3: device 4, Example 4: device 5, Example 5: device 6, Example 6: device 7, Example 7: device 8, Example 8: device 9), and by using the obtained devices, CRP, an enzyme labeled anti-CRP antibody, and a substrate were sequentially added dropwise thereto to perform an immunoassay by a sandwich method. Specific procedures are shown below.

(Immobilization of Detection substance)

An anti-CRP antibody was immobilized on each of the substrates according to the method described above in Experimental Example 1.

(Fabrication of Device)

Each substrate and an absorbent pad were combined according to the method described above in Experimental Example 1. According to the above-described procedures, the device 3 employing the sheet substrate 1 obtained in Production Example 1, the device 4 employing the sheet substrate 2 obtained in Production Example 2, the device 5 employing the sheet substrate 3 obtained in Production Example 3, the device 6 employing the sheet substrate 4 obtained in Production Example 4, the device 7 employing the sheet substrate 5 obtained in Production Example 5, the device 8 employing the sheet substrate 6 obtained in Production Example 6, and the device 9 employing the sheet substrate 7 obtained in Production Example 7 were obtained.

(Immunoassay)

An immunoassay was performed according to the following procedure by using each of the devices 3 to 9.
Unless stated otherwise, each aqueous solution was dropped onto the end part of the substrate of each example (at a position 5 mm away from the edge on the opposite side of the side superposed with the absorbent pad).

1. 10 $\mu$L of an aqueous solution for washing (0.05 (v/v)% Triton X-100 / Tris-buffered saline (TBS)) was dropped to wash surplus antibodies on the substrate of each example.
2. 10 $\mu$L of an aqueous solution obtained by diluting CRP (manufactured by BBI Solutions OEM, Ltd.) with 0.05 (v/v)% Triton X-100 / TBS (CRP concentration 10 ug/mL) or an aqueous solution including no CRP (0.05 (v/v)% Triton X-100 / TBS) was dropped, and the substrates were let stand for 3 minutes.
3. 10 $\mu$L of an alkaline phosphatase-labeled anti-CRP antibody solution (antibody concentration about 20 ug/mL) was dropped, and the substrates were let stand for 1 minute. This operation was repeated two more times.
4. 10 $\mu$L of an aqueous solution for washing (0.05 (v/v)% Triton X-100 / Tris-buffered saline (TBS)) was dropped to wash surplus labeled antibodies on the substrate of each example.
5. 10 $\mu$L of a BCIP-NBT solution prepared by mixing a BCIP-NBT color-developing stock solution (manufactured by Nacalai Tesque, Inc.) with a Tris-HCl buffer solution (manufactured by Nacalai Tesque, Inc.) at a volume ratio of 1:100, was dropped, and the substrates were let stand for 3 minutes.
6. 10 $\mu$L of an aqueous solution for washing (0.05 (v/v)% Triton X-100 / TBS) was dropped to wash surplus antigens, the substrate, and the product material on the substrate.
7. Images of the substrate of each example after washing were captured by using a digital camera (manufactured by Casio Computer Co., Ltd., EXILIM EX-100F).

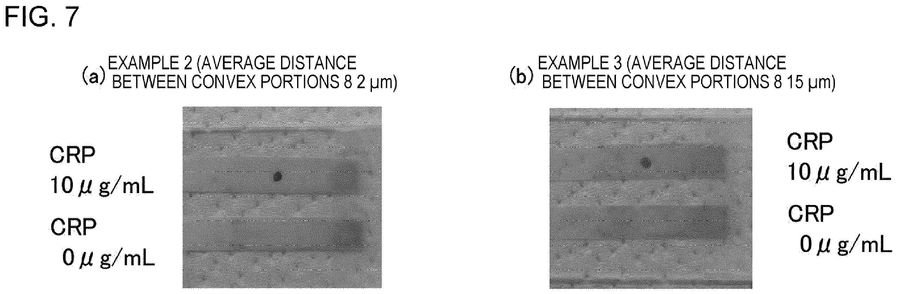

Among the images of the above-described 7., images of the device 3 (Example 2) and the device 4 (Example 3) are shown in Fig. 7(a) and Fig. 7(b), respectively.
From Fig. 7(a) and Fig. 7(b), purple coloration was identified on each substrate as a result of a reaction between the enzyme (alkaline phosphatase) introduced onto the substrate in the procedure 3. and the substrate (BCIP-NBT) introduced onto the substrate in the procedure 5. In both the devices of Examples 2 and 3, clear coloration was observed from the detection zone (region where the antibody was applied) of the substrate with which a sample containing the antigen was measured. Furthermore, coloration was hardly identified in the regions other than the detection zone (regions where the antibody was not applied) of the substrate with which a sample containing the antigen was measured, and the substrate with which a sample containing no antigen was measured.

(Observation of SEM image at antibody-applied portion)

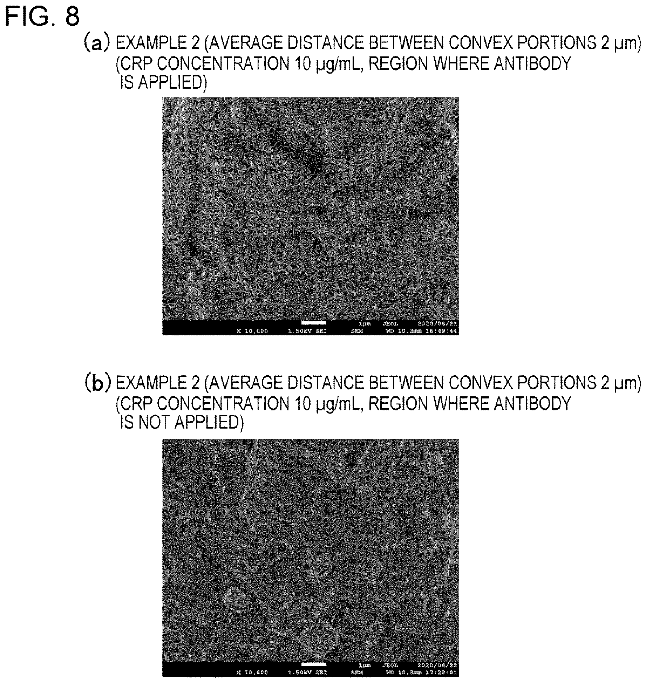

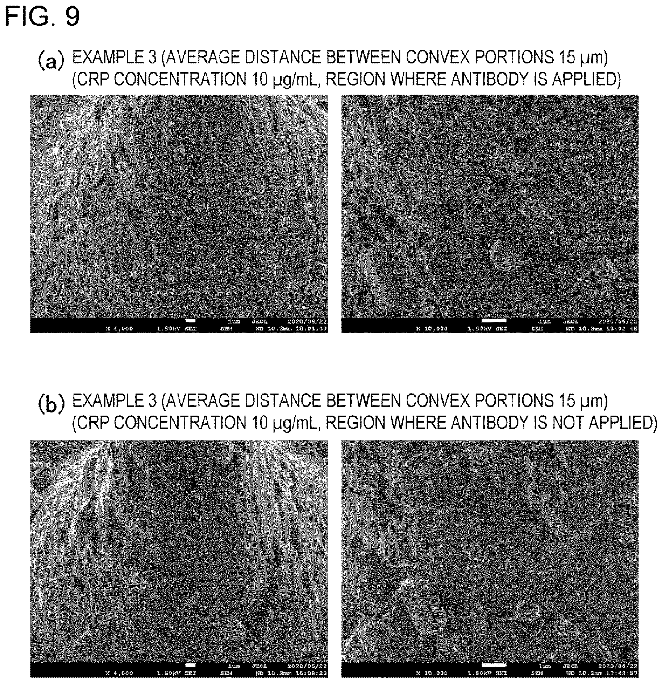

Fig. 8(a), Fig. 8(b), Fig. 9(a), and Fig. 9(b) are diagrams showing the SEM images obtained by performing immunoassays according to the procedures described above in Example 2 (Fig. 8(a) and Fig. 8(b)) and Example 3 (Fig. 9(a) and Fig. 9(b)) and then observing the detection zone 105 (in the diagrams, "region where antibody is applied") and a region other than the detection zone 105 (in the diagrams, "region where antibody is not applied") in each substrate. From Fig. 8(a) and Fig. 9(a), it was verified that in the detection zone 105 (region where antibody is applied) of each example, the coloring substance having a size of several tens of nanometers to several hundreds of micrometers was closely deposited on the fine rough structure A. On the other hand, in the region other than the detection zone 105 (region where antibody is not applied), deposition of the coloring substance having a size of several tens of nanometers to several hundreds of micrometers was hardly observed.

(Calculation of ΔRGB in region where antibody was applied)

ΔRGB was calculated according to the method described above in Experimental Example 1. The calculation results of ΔRGB for each substrate are shown in Table 3.

[Table 3]

Table 3

|  | Example 2 | Example 3 | Example 4 | Example 5 | Example 6 | Example 7 | Example 8 |
|---|---|---|---|---|---|---|---|
| Sheet substrate | Production Example 1 | Production Example 2 | Production Example 3 | Production Example 4 | Production Example 5 | Production Example 6 | Production Example 7 |
| ΔRGB | 110.1 | 74.0 | 74.3 | 60.6 | 50.4 | 47.4 | 31.7 |

From Table 3, in all of the devices employing the sheet substrate having the fine rough structure A, ΔRGB was higher compared to the devices employing the membrane substrate of Comparative Example 1 (Table 2). Among them, the ΔRGB was even higher in the devices employing the sheet substrate having the fine rough structure A of Example 2.

(Experimental Example 3: Examples 9 and 10 and Comparative Example 2)

In Examples 9 and 10 and Comparative Example 2, devices were produced by immobilizing a detection substance on the above-mentioned sheet substrates 1 and 2 and the above-mentioned membrane substrate 1, respectively (Example 9: device 10, Example 10: device 11, Comparative Example 2: device 12), and an immunoassay using chemiluminescence caused by a luminol reaction was performed by using the obtained devices.

(Immobilization of Detection substance)

An anti-CRP antibody was immobilized on each of the substrates according to the method described above in Experimental Example 1.

(Fabrication of Device)

Each substrate and an absorbent pad were combined according to the method described above in Experimental Example 1. Through the above-described procedures, a device 10 employing the sheet substrate 1 obtained in Production Example 1 and a device 11 employing the sheet substrate 2 obtained in Production Example 2 were obtained.
On the other hand, a device 12 was obtained by immobilizing a detection substance on a membrane substrate 1 formed from a nitrocellulose membrane.

(Immunoassay)

An immunoassay was performed according to the following procedure by using each of the devices 10 to 12.

Unless stated otherwise, each aqueous solution was dropped onto the end part of the substrate of each example (at a position 5 mm away from the edge on the opposite side of the side superposed with the absorbent pad).

1. 20 µL of an aqueous solution for washing (0.05 (v/v)% Triton X-100 / TBS) was dropped to wash surplus antibodies on the substrate of each example.

2. 20 µL of an aqueous solution obtained by diluting CRP (manufactured by BBI Solutions OEM, Ltd.) with 0.05 (v/v)% Triton X-100 / TBS (CRP concentration 10 µg/mL) or an aqueous solution including no CRP (0.05 (v/v)% Triton X-100 / TBS) was dropped, and the substrates were let stand for 3 minutes.

3. 20 µL of a horseradish peroxidase (HRP)-labeled anti-CRP antibody (antibody concentration about 0.2 µg/mL) was dropped, and the substrates were let stand for 2 minutes. This operation was repeated once more.

4. 20 µL of an aqueous solution for washing (0.05 (v/v)% Triton X-100 / TBS) was dropped to wash surplus labeled antibodies on the substrate of each example.

5. A substrate solution was prepared by mixing equal amounts of a Stable Peroxide Solution and a Luminol/Enhancer Solution in SuperSignal West Femto Maximum Sensitivity Substrate (trade name, manufactured by Thermo Fisher Scientific, Inc.), and 20 µL was dropped thereon.

6. After the dropping, chemiluminescence images obtained after 1 minute and 45 seconds were captured using a chemiluminescence measuring apparatus (manufactured by Bio-Rad Laboratories, Inc., ChemiDoc XRS Plus System).

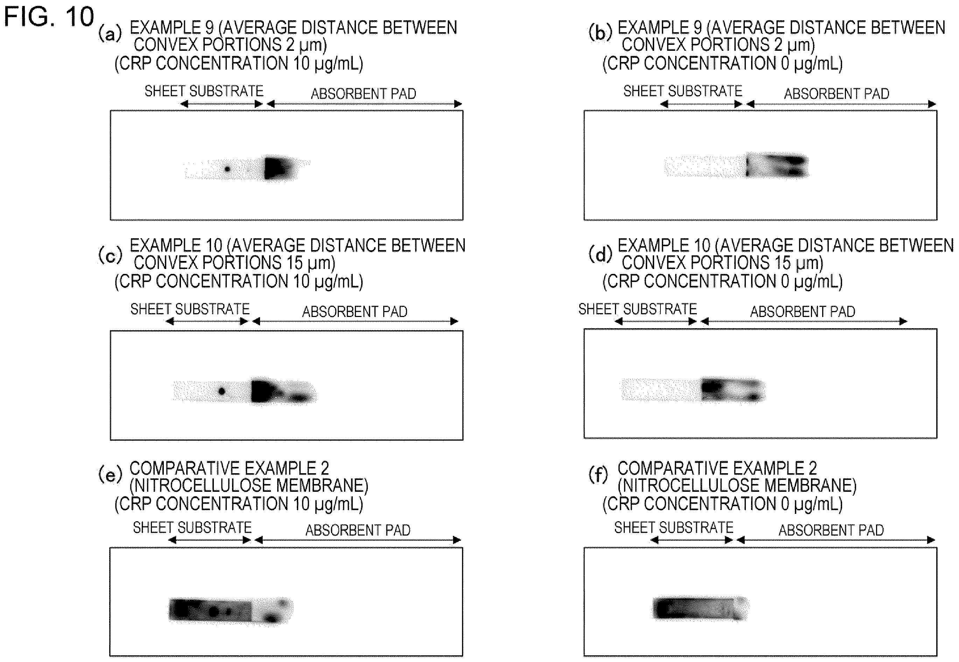

Chemiluminescence images of each substrate after the measurement are shown in Fig. 10(a) to Fig. 10(f). In each of these figures, a rectangular frame indicates the frame of the image. In both Example 9 (Fig. 10(a) and Fig. 10(b)) and Example 10 (Fig. 10(c) and Fig. 10(d)), chemiluminescence was identified (Fig. 10(a) and Fig. 10(c)) in the detection zone 105 (region where the antibody was applied) of the sheet substrates. Furthermore, luminescence was hardly identified from the regions other than the detection zone 105 (regions where the antibody was not applied) in the sheet substrate and from the substrates with which a sample containing no antigen was measured (Fig.10(b) and Fig. 10 (d)).

On the other hand, in Comparative Example 2 (Fig. 10(e) and Fig. 10(f)), with regard to the device 12 employing the membrane substrate 1, luminescence was observed not only in the detection zone 105 (region where the antibody was applied) of the substrate with which a sample containing the antigen was measured but also in the regions other than the detection zone 105 (regions where the antibody was not applied), and the substrates with which a sample containing no antigen was not measured.

From the results described above, it was found that in the devices 10 and 11 employing the sheet substrates having the fine rough structure A of Examples 9 and 10, since a detection substance and a coloring substance can be efficiently immobilized on the convex portions 8, a detection signal (chemiluminescence), which is a change in the optical characteristics caused by an enzymatic reaction, is easily recognized in the detection zone 105, that is, the S/N ratio is increased, as compared with the device 12 employing the membrane substrate of Comparative Example 2, and measurement with high sensitivity and high accuracy is enabled.

This patent application claims priority on the basis of Japanese Patent Application No. 2021-052926, filed on March 26, 2021, the disclosure of which is incorporated herein in its entirety by reference.

REFERENCE SIGNS LIST

4 diameter
5 distance
6 height
8 convex portion
9 flat portion
10 bottom surface
11 bottom surface
14 diameter
16 height
18 convex portion
19 center
20 straight line

100 test device
101 sheet substrate
103 flow path
105 detection zone
d direction of transfer
R concave portion

**Claims**

1. A test device comprising:

   a flow path provided on one surface of a sheet-shaped resin substrate for transferring a liquid sample by a capillary phenomenon;
   a detection zone provided in a portion of the flow path; and
   a fine rough structure A having a plurality of convex portions, the fine rough structure A being provided at least in the detection zone and formed integrally with the resin substrate,
   wherein in the detection zone, a detection substance that specifically binds to a substance to be detected in the liquid sample is immobilized on a surface of the fine rough structure A, and
   the test device is configured to detect the substance to be detected by sensing a change in optical characteristics of the detection zone occurring due to an enzymatic reaction.

2. The test device according to Claim 1, wherein the detection substance is an antibody against the substance to be detected.

3. The test device according to Claim 1 or 2, wherein the change in the optical characteristics is a color change in the detection zone.

4. The test device according to Claim 3, wherein the color change is visually identifiable.

5. The test device according to Claim 1 or 2, wherein the change in the optical characteristics is chemiluminescence, bioluminescence, or fluorescence in the detection zone.

6. The test device according to any one of Claims 1 to 5, wherein in the detection zone, the convex portions have a shape of a columnar body, a pyramidal body, or a frustum.

7. The test device according to any one of Claims 1 to 6, wherein in the detection zone, the shape of the convex portions is a conical body, and the detection substance is immobilized on a lateral face of the conical body.

8. The test device according to any one of Claims 1 to 7, wherein in the detection zone, the plurality of convex portions are planarly arranged in a lattice pattern.

9. The test device according to any one of Claims 1 to 8, wherein the convex portions have a bottom surface with a diameter of equal to or more than 10 um and equal to or less than 1000 um.

10. The test device according to any one of Claims 1 to 9, wherein the convex portions have a height of equal to or more than 10 um and equal to or less than 500 um.

11. The test device according to any one of Claims 1 to 10, wherein at least in the detection zone, a fine rough structure B is formed on a surface of the convex portions, and
    an arithmetic mean roughness Ra of a roughness curve of the convex portions on which the fine rough structure B is formed is equal to or more than 0.005 um and equal to or less than 1 um.

12. The test device according to any one of Claims 1 to 11, wherein in one cross-section, a plurality of the convex portions have:

    a first region located on one side of each convex portion from a center of the convex portion in a width direction of the convex portion; and

a second region located on other side of the convex portion from the center of the convex portion in the width direction of the convex portion, and

in the one cross-section, at least one of an outer edge of the first region and an outer edge of the second region of the convex portion has a concave portion.

13. The test device according to any one of Claims 1 to 12, which is used for an enzyme immunoassay method.

14. A test method of using the test device according to any one of Claims 1 to 13, the test method comprising:

a step of introducing the liquid sample into an upstream side of the detection zone to guide the liquid sample to the detection zone by a capillary phenomenon, and trapping the substance to be detected in the fine rough structure A by a specific interaction between the substance to be detected and the detection substance;

after the step of trapping, a step of introducing a liquid including a labeled antibody that specifically binds to the substance to be detected into the detection zone, and trapping the labeled antibody in the fine rough structure A in which the substance to be detected is trapped;

after the step of trapping the labeled antibody, a step of introducing, into the detection zone, a compound that causes a change in optical characteristics in the detection zone by reacting with the labeled antibody; and

a step of detecting or quantitatively determining the substance to be detected by sensing the change in the optical characteristics in the detection zone.

FIG. 1

FIG. 2

(a)

(b)

FIG. 3

(a) PRODUCTION EXAMPLE 1

(b) PRODUCTION EXAMPLE 2

(c) PRODUCTION EXAMPLE 3

(d) PRODUCTION EXAMPLE 4

(e) PRODUCTION EXAMPLE 5

(f) PRODUCTION EXAMPLE 6

FIG. 4

FIG. 5

## FIG. 6

(a) EXAMPLE 1 (AVERAGE DISTANCE
BETWEEN CONVEX PORTIONS 2 μm)
(CRP CONCENTRATION 10 μg/mL, n = 3)

(b) EXAMPLE 1 (AVERAGE DISTANCE
BETWEEN CONVEX PORTIONS 2 μm)
(CRP CONCENTRATION 0 μg/mL, n = 3)

(c) COMPARATIVE EXAMPLE 1
(NITROCELLULOSE MEMBRANE)
(CRP CONCENTRATION 10 μg/mL, n = 3)

(d) COMPARATIVE EXAMPLE 1
(NITROCELLULOSE MEMBRANE)
(CRP CONCENTRATION 0 μg/mL, n = 3)

EP 4 317 976 A1

FIG. 7

(a) EXAMPLE 2 (AVERAGE DISTANCE BETWEEN CONVEX PORTIONS 8 2 μm)

CRP
10 μ g/mL

CRP
0 μ g/mL

(b) EXAMPLE 3 (AVERAGE DISTANCE BETWEEN CONVEX PORTIONS 8 15 μm)

CRP
10 μ g/mL

CRP
0 μ g/mL

# FIG. 8

(a) EXAMPLE 2 (AVERAGE DISTANCE BETWEEN CONVEX PORTIONS 2 μm)
(CRP CONCENTRATION 10 μg/mL, REGION WHERE ANTIBODY
IS APPLIED)

(b) EXAMPLE 2 (AVERAGE DISTANCE BETWEEN CONVEX PORTIONS 2 μm)
(CRP CONCENTRATION 10 μg/mL, REGION WHERE ANTIBODY
IS NOT APPLIED)

# FIG. 9

(a) EXAMPLE 3 (AVERAGE DISTANCE BETWEEN CONVEX PORTIONS 15 μm)
(CRP CONCENTRATION 10 μg/mL, REGION WHERE ANTIBODY IS APPLIED)

(b) EXAMPLE 3 (AVERAGE DISTANCE BETWEEN CONVEX PORTIONS 15 μm)
(CRP CONCENTRATION 10 μg/mL, REGION WHERE ANTIBODY IS NOT APPLIED)

# FIG. 10

(a) EXAMPLE 9 (AVERAGE DISTANCE BETWEEN CONVEX PORTIONS 2 μm) (CRP CONCENTRATION 10 μg/mL)

(b) EXAMPLE 9 (AVERAGE DISTANCE BETWEEN CONVEX PORTIONS 2 μm) (CRP CONCENTRATION 0 μg/mL)

(c) EXAMPLE 10 (AVERAGE DISTANCE BETWEEN CONVEX PORTIONS 15 μm) (CRP CONCENTRATION 10 μg/mL)

(d) EXAMPLE 10 (AVERAGE DISTANCE BETWEEN CONVEX PORTIONS 15 μm) (CRP CONCENTRATION 0 μg/mL)

(e) COMPARATIVE EXAMPLE 2 (NITROCELLULOSE MEMBRANE) (CRP CONCENTRATION 10 μg/mL)

(f) COMPARATIVE EXAMPLE 2 (NITROCELLULOSE MEMBRANE) (CRP CONCENTRATION 0 μg/mL)

# FIG. 11

(a)

(b)

FIG. 12
(a)

d

30 μm

30 μm

18

14

(b)

18

30 μm

30 μm

10 μm

# FIG. 13

PRODUCTION EXAMPLE 7

## INTERNATIONAL SEARCH REPORT

International application No.

PCT/JP2022/014157

### A. CLASSIFICATION OF SUBJECT MATTER

*G01N 33/543*(2006.01)i
FI: G01N33/543 521

According to International Patent Classification (IPC) or to both national classification and IPC

### B. FIELDS SEARCHED

Minimum documentation searched (classification system followed by classification symbols)

G01N33/543

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP 2019-100714 A (TOYO SEIKAN GROUP HOLDINGS, LTD.) 24 June 2019 (2019-06-24) see entire text, all drawings, in particular, claims, paragraphs [0017]-[0032], [0039]-[0063], fig. 1-11, etc. | 1-10, 13-14 |
| Y | JP 2006-138776 A (SEKISUI CHEMICAL CO., LTD.) 01 June 2006 (2006-06-01) see entire text, in particular, claims etc. | 1-10, 13-14 |
| X | IKAMI M et al., IMMUNO-PILLAR CHIP: A NEW PLATFORM FOR RAPID AND EASY-TO-USE IMMUNOASSAY, Lab on a Chip, 2010, vol. 10, pp. 3335-3340 see entire text, all drawings, in particular, Abstract, Fabrication of immuno-pillar chip, etc. | 1-10, 13-14 |
| A | WO 2020/217635 A1 (DENKA CO., LTD.) 29 October 2020 (2020-10-29) see entire text, all drawings, etc. | 1-14 |
| A | JP 2014-510925 A (ORTHO-CLINICAL DIAGNOSTICS, INC.) 01 May 2014 (2014-05-01) see entire text, all drawings, etc. | 1-14 |

☐ Further documents are listed in the continuation of Box C.    ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **01 June 2022** | **14 June 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

| Patent document cited in search report | | | Publication date (day/month/year) | Patent family member(s) | Publication date (day/month/year) |
|---|---|---|---|---|---|
| JP | 2019-100714 | A | 24 June 2019 | (Family: none) | |
| JP | 2006-138776 | A | 01 June 2006 | (Family: none) | |
| WO | 2020/217635 | A1 | 29 October 2020 | KR 10-2021-0142702 A<br>see entire text, all drawings, etc. | |
| JP | 2014-510925 | A | 01 May 2014 | US 2012/0258479 A1<br>see entire text, all drawings, etc.<br>WO 2012/138701 A1<br>EP 2694967 A1 | |

Form PCT/ISA/210 (patent family annex) (January 2015)

34

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005502363 W
- JP 2021052926 A